# EUROPÄISCHE PATENTSCHRIFT

(11) **EP 2 735 290 B1**
(45) Veröffentlichungstag und Bekanntmachung des Hinweises auf die Patenterteilung: **17.04.2019**
(21) Anmeldenummer: 13194452.2
(22) Anmeldetag: 26.11.2013
(51) Int. Cl.: A61N 2/00, A61F 5/03, A61F 7/00, A61N 1/36, A61N 1/04, A61F 5/01, A61F 2/50, A61F 2/78, A61F 2/80

(54) **Prothese oder Orthese**
Prosthesis or orthosis
Prothèse ou orthèse

(30) Priorität: 26.11.2012 DE 102012023071
(43) Veröffentlichungstag der Anmeldung: 28.05.2014
(73) Patentinhaber: Pohlig GmbH, 83278 Traunstein (DE)
(72) Erfinder: Pohling, Kurt, 83278 Traunstein (DE); Schäfer, Michael, 83278 Traunstein (DE)
(74) Vertreter: Pfenning, Meinig & Partner mbB

(56) Entgegenhaltungen:
- WO-A1-93/15696
- WO-A1-2012/069923
- WO-A2-2007/102156
- FR-A- 1 548 465
- US-A- 4 923 475
- US-A- 5 108 456
- US-A1- 2002 099 450
- US-A1- 2011 118 853

## Beschreibung

Die Erfindung betrifft eine Prothese oder Orthese für den Ersatz von äußeren Körperteilen oder Gliedmaßen, beispielsweise nach Amputationen, bzw. zum Stabilisieren, Entlasten, Ruhigstellen und Führen von Gliedmaßen oder des Rumpfs oder zum Korrigieren von Fehlstellungen von Gliedmaßen oder des Rumpfs.

Bei der Herstellung von Prothesen oder Orthesen ist die möglichst genaue Anpassung des Schafts der Prothese oder Orthese an einen Stumpf bzw. an eine Gliedmaße ein zentrales Problem. Im Fall einer Prothese erhält man ohne eine möglichst genaue Anpassung des Schafts keine stabile Verbindung zwischen dem Stumpf und der Prothese, wodurch die Kraftübertragung auf die Prothese bei einer Bewegung des Stumpfes verschlechtert ist und Verletzungen am Stumpf vermehrt auftreten können. Bei einer Orthese ist der Therapieerfolg stark vom Sitz der Orthese an dem zu therapierenden Körperteil abhängig. Bei der Korrektur von Fehlstellungen von Gliedmaßen kommt es beispielsweise oft darauf an dem zu behandelnden Körperteil einen definierten Bewegungsablauf aufzuzwingen. Dies ist jedoch nur möglich, wenn die Orthese optimal an dem Körperteil sitzt.

Eine Möglichkeit die Verbindung zwischen dem Körperteil und der Prothese oder Orthese zu verbessern ist, den Schaft enger zu gestalten. Jedoch können durch einen zu eng gestalteten Schaft unangenehme Druckschmerzen auftreten, da die Empfindlichkeit gegenüber Druck im Allgemeinen über verschiedene Bereiche des Körperteils variiert.

Ferner kann sich selbst bei einmalig angepasstem Schaft der Umfang des Körperteils mit der Zeit aufgrund von tageszeitlichen oder langfristigen Veränderungen, z.B. tageszeitliche An- und Abschwellung, Gewichtszunahme und -abnahme oder Muskelschwund, sodass ein optimaler Sitz der Prothese oder Orthese nicht mehr gewährleistet ist.

Die Druckschrift US 5 108 456 A beschreibt eine prothetische Vorrichtung für einen Beinstumpf, welche ein Prothesenteil mit einer Aussparung umfasst, wobei die Aussparung durch Seitenwände aus einem starren Material gebildet ist. In dieser Aussparung befindet sich eine herausnehmbar gestaltete Fassung aus einem flexiblen Material zur Aufnahme des Beinstumpfs. An der Außenseite der Fassung sind aufblasbare Kammern befestigt, welche gegen die Innenseite der starren Seitenwände des Prothesenteils wirken. Hierdurch werden die Seitenwände der Fassung nach innen gedrückt, um den Beinstumpf zu festzuhalten.

Die Druckschrift WO 2012/069923 A1 offenbart eine Lendenstützvorrichtung, welche ein Basiselement und ein Polsterelement, das an dem Basiselement befestigt ist, beinhaltet, wobei das Polsterelement eine Vielzahl von Druckelementen, die sich von einem vorderen Abschnitt des Basiselements erstrecken. Die Lendenstützvorrichtung ist geeignet, um zwischen der Kleidung eines Benutzers und dem Rücken eines Benutzers positioniert zu werden, ohne eine Befestigung an der Kleidung des Benutzers oder dem Rücken des Benutzers, so dass die Vielzahl von Druckelementen mit mehreren diskreten Stellen auf dem Rücken des Benutzers in Eingriff sind.

Die Druckschrift US 4 923 475 A offenbart eine Prothese mit einer Fassung zur Aufnahme eines Stumpfes und einer Vielzahl von aufblasbaren Kammern mit einem Mittel zum Variieren der Drücke darin. Die statischen Drücke in den Kammern sind unabhängig voneinander und können vom Benutzer reguliert werden.

Die Druckschrift WO 93/15696 offenbart eine Beinprothese mit einer an der Schaftwandung angeordneten Backe sowie einem im Stumpfendbereich angeordneten aufblähbaren Kissen. Hierdurch ist ein auf schonende Weise dauerhafter dosierbarer Stumpfendkontakt herstellbar, wie er aus orthopädischen und auch lymphangiologischen Gesichtspunkten wünschenswert ist.

Die Druckschrift FR 1 548 465 A offenbart ein Verfahren und eine Vorrichtung zum Realisieren der Kompression von Gliedmaßenprothesen. Hierbei ist die Innenseite der Prothese mit einer Kammer versehen, welche über ein Ventil von außen aufblasbar ist.

Die Druckschrift US 2002/0099450 A1 offenbart ein dynamisches Befestigungssystem mit variabler Geometrie für einen Prothesenschaft. Das System verfügt über flüssigkeitsgefüllte Blasen, deren Volumina sich automatisch regulierbaren, um einen kontinuierlich sicheren Sitz des Prothesenschafts zu gewährleisten. Das Befestigungssystem ist in der Lage das Potenzial für Gewebeläsionen zu reduzieren.

Ausgehend von den oben beschriebenen Problemen ist es daher die Aufgabe der vorliegenden Erfindung eine Prothese oder Orthese bereitzustellen, die eine stabilere, insbesondere eine rotationsstabilere, Verbindung zwischen einem Körperteil und der Prothese oder Orthese ermöglicht und dabei unangenehme Druckgefühle vermeidet.

Die Aufgabe wird durch eine Prothese oder Orthese nach Anspruch 1 gelöst. Vorteilhafte Ausgestaltungen der erfindungsgemäßen Prothese oder Orthese sind in den abhängigen Ansprüchen beschrieben.

Die erfindungsgemäße Prothese oder Orthese weist einen einschaligen oder mehrschaligen Schaft zur Aufnahme eines Körperteils auf, wobei der Schaft eine innere Wandung aufweist, die einen Körperteilaufnahmebereich begrenzt. Unter dem Körperteilaufnahmebereich ist die Ausnehmung des Schaftes zu verstehen, die von der inneren Wandung begrenzt wird. Der Schaft ist mit dieser Ausnehmung an ein Körperteil ansteckbar oder anlegbar, wobei die innere Wandung am Körperteil anliegt.

Die innere Wandung schließt den Körperteilaufnahmebereich in mindestens einer Ebene vollständig um. Die innere Wandung der erfindungsgemäßen Prothese oder Orthese weist außerdem mindestens zwei Ausstülpungen auf.

Die derart ausgestaltete erfindungsgemäße Prothese oder Orthese ermöglicht eine stabilere Verbindung zwischen einem Körperteil, beispielsweise einem Stumpf bzw. einer Gliedmaße oder dem Rumpf, und der Prothese oder Orthese. Die Ausstülpungen der inneren Wandung erhöhen insbesondere die Rotationsstabilität der Prothese oder Orthese, ohne dass der Schaft über die Maßen eng gestaltet werden muss. Hierdurch kann ein Druck auf druckempfindliche Bereiche des entsprechenden Körperteils vermieden werden.

Die Ausstülpungen weisen dabei vorzugsweise eine Tiefe von 5mm +/- 3mm und einen mittleren Flächendurchmesser von 50 mm +/- 10 mm auf. Unter der Tiefe der Ausstülpung wird die maximale Ausdehnung der Ausstülpung radial zur Längsachse des Schafts gemessen von der Oberfläche der inneren Wandung verstanden. Unter dem Flächendurchmesser der Ausstülpung wird die Ausdehnung der Ausstülpung entlang der Oberfläche der inneren Wandung verstanden.

In einer weiteren vorteilhaften Ausgestaltung der Erfindung weist die innere Wandung mindestens vier Ausstülpungen auf, wobei die Ausstülpungen derart angeordnet sind, dass in einer Projektion der inneren Wandung auf eine Ebene senkrecht zu einer Längsachse des Schafts die Ausstülpungen lediglich bereichsweise überlappen. Diese Anordnung von vier Ausstülpungen, in der die Ausstülpungen bezüglich einer Ebene senkrecht zur Längsachse, also bezüglich der Richtung der Längsachse, versetzt zueinander angeordnet sind, verbessert die Verbindungsstabilität, insbesondere die Rotationsstabilität zwischen der Prothese oder Orthese und dem entsprechenden Körperteil.

Alternativ kann die innere Wandung auch eine Vielzahl von Ausstülpungen aufweisen oder mit einer Vielzahl von Ausstülpungen oder vollständig mit Ausstülpungen überdeckt sein. Dies vergrößert die Oberfläche der inneren Wandung, wodurch der Sitz der Prothese oder Orthese am entsprechenden Körperteil weiter verbessert wird.

Die Ausstülpungen können als Auskragungen oder als Vertiefungen ausgebildet sein. Es ist auch denkbar, dass ein Teil der Ausstülpungen als Auskragungen ausgebildet ist und ein weiterer Teil der Ausstülpungen als Vertiefungen ausgebildet ist. In den Ausstülpungen, die als Vertiefungen ausgebildet sind, kann sich ein Unterdruck einstellen, wodurch wiederum die Haftung der Prothese oder Orthese am entsprechenden Körperteil erhöht wird. Hierzu können auch Ein-Weg-Ventile in den Vertiefungen angeordnet sein, mittels denen Luft aus den Vertiefungen absaugbar ist. Die Ausstülpungen, die als Auskragungen ausgebildet sind können zusätzlich als stimulierende Druckpunkte, z.B. Akupressoren, wirken.

Der Schaft der Prothese oder Orthese kann vorzugsweise zylinderförmig oder konisch gestaltet sein. Die Ausstülpungen können kuppelförmig, kantig, konvex, konkav, sternförmig, oval oder eckig ausgebildet sein.

Um die Prothese oder Orthese an kurzfristige oder langfristige Veränderungen des Umfangs des Körperteils optimal anpassen zu können, ist es besonders vorteilhaft, wenn die Tiefe und/oder die Dicke bzw. die Gesamtgröße der Ausstülpungen pneumatisch oder hydraulisch oder mechanisch variierbar ist. Hierzu kann im Schaft ein System miteinander kommunizierender Röhren zur hydraulischen oder pneumatischen oder mechanischen Regulierung der Tiefe und/oder Dicke bzw. der Gesamtgröße der Ausstülpungen integriert sein. Mit einem solchen System kann das Schaftvolumen auch derart geregelt werden, dass bei punktuell starkem Druck ausgehend von einer ersten Ausstülpung, beispielsweise beim Sitzen mit einer Beinprothese oder -orthese, dieser auf andere Ausstülpungen, die mit der ersten Ausstülpung über das Röhrensystem verbunden sind, verlagert werden kann.

Alternativ ist es auch möglich, dass zur Regulierung der Tiefe und/oder der Dicke bzw. der Gesamtgröße der Ausstülpungen ein Wechseldrucksystem vorgesehen ist, womit die Tiefe und/oder Dicke bzw. die Gesamtgröße von einzelnen oder mehreren Ausstülpungen abwechselnd erhöht oder verringert werden kann. Ein derartiges Wechseldrucksystem ist insbesondere zur Vermeidung von dauerhaften Druckstellen und sich daraus entwickelnden Geschwüren vorteilhaft.

Eine weitere vorteilhafte Ausgestaltung sieht vor, dass mindestens eine der Ausstülpungen mindestens eine Kammer oder einen Hohlraum aufweist. Diese Kammer oder dieser Hohlraum ist geeignet zusätzliche Instrumente oder Mittel zur Überwachung oder Behandlung des Körperteils aufzunehmen. Ebenso können auch alle Ausstülpungen eine oder mehrere Kammern aufweisen.
Beispielsweise kann in mindestens einer Kammer ein Permanentmagnet oder ein Elektromagnet zur Erzeugung eines statischen oder eines gepulsten Magnetfeldes angeordnet sein. Gepulste elektromagnetische Felder (PEMF) sollen bei bestimmten Frequenzen den Organismus bioenergetisch positiv beeinflussen und die Durchblutung fördern, um den Zellstoffwechsel anzuregen.

Eine weitere Möglichkeit sieht vor, dass mindestens eine Kammer ein Piezoelement zur elektrischen und/oder mechanischen Stimulation des Körperteils beinhaltet.

In mindestens einer Kammer mindestens einer Ausstülpung können alternativ auch Medikamente deponiert werden, wobei die Medikamente über eine oder mehrere Öffnungen oder eine Perforation in der Kammer an eine Hautoberfläche des Körperteils abgebbar sind. Gleichermaßen ist auch die Einrichtung mehrerer verschiedener Medikamentendepots in verschiedenen Kammern einer oder mehrerer Ausstülpungen möglich, wenn mehr als ein Medikament verabreicht werden soll.

In einer weiteren vorteilhaften Ausgestaltung der Erfindung sind eine oder mehrere Ausstülpungen zur Beheizung und/oder Kühlung des Schafts eingerichtet. Durch die Zufuhr von Wärme werden beispielsweise die Gefäße erweitert und die Durchblutung gefördert. Zur Beheizung und/oder Kühlung kann der Schaft von einem Schlauchsystem durchzogen sein, das mit den Ausstülpungen wärmeleitend verbunden ist. Alternativ können eine oder mehrere Kammern einer oder mehrerer Ausstülpungen als Wärmekammer zur Erwärmung von arteriellem Blut an einer zentralen Einströmstelle des arteriellen Blutes (z.B. Arteria femoralis) eingerichtet sein, um das peripher strömende Blut zu erwärmen und so beispielsweise dem oft beklagten Kältegefühl im Beinstumpf entgegenzuwirken.

Ferner kann mindestens eine Kammer ein Vibrationselement beinhalten. Das Vibrationselement überträgt mechanische Schwingungen auf das von der Prothese oder Orthese aufgenommene Körperteil. Dies fördert die Relaxierung der Muskulatur und die Durchblutung des Körperteils und reduziert Muskelverspannungen.

In einer weiteren möglichen Ausgestaltung der Erfindung kann mindestens eine Kammer mindestens einer Ausstülpung eine Füllung mit Feuchtigkeit aufsaugendem Material zur Dehydrierung des in der Regel sehr feuchten Innenschaftklimas aufweisen. Dieses Feuchtigkeit aufsaugende Material kann beispielsweise in Form von Pads in eine oder mehrere Kammern einer oder mehrerer Ausstülpungen eingesetzt sein. Alternativ kann in einer oder mehreren Kammern einer oder mehrerer Ausstülpungen auch ein elektronisch und/oder mechanisch wirkendes Entfeuchtungselement angeordnet sein.

In einer weiteren vorteilhaften Ausgestaltung der Erfindung weist mindestens eine Kammer in mindestens einer Ausstülpung mindestens ein Messgerät zur Messung von Körperzuständen, eines Blutdrucks, einer Strömungsgeschwindigkeit von Blut und/oder einer Körpertemperatur auf.

Darüberhinaus ist für Langzeitdiagnosen die Abfrage von bionischen Daten interessant. Hierzu kann mindestens eine Kammer mindestens einer Ausstülpung ein Messgerät zur Erfassung von bionischen Daten wie z.B. eines Oberflächendruckes an definierten Messpunkten, einer Schaftinnentemperatur und/oder eines Feuchtigkeitsgrades aufweisen.

In einer weiteren vorteilhaften Ausgestaltung der Erfindung kann mindestens in einer Ausstülpung eine Elektrode zur Abnahme von myoelektrischen Impulsen zur Steuerung von mechanischen Knie-, Fuß-, Hand- oder Ellenbogengelenken angebracht sein.

Zur Fern-Überwachung der Rehabilitationstherapie oder der Tragezeit kann mindestens eine der Ausstülpungen auch mindestens einen Temperatur- und/oder Bewegungslogger aufweisen.

Die Ausstülpungen und die innere Wandung weisen vorteilhafterweise das gleiche Material auf. Je nach Versorgungsziel kann die innere Wandung beispielsweise ein elastisches Material, ein Material mit einem weichen Shore-Grad oder ein rigides Material aufweisen, in das die Ausstülpungen integriert sind.

Die Oberfläche der Ausstülpungen kann glatt, rau, adhäsiv, mit einem Relief und/oder mit Noppen versehen sein. Die Noppen können vollständig mit Material gefüllt oder hohl gestaltet sein.

Es ist ebenso denkbar, dass die Ausstülpungen und die innere Wandung unterschiedliche Materialien aufweisen. In diesem Fall können die Ausstülpungen vorzugsweise auf eine Oberfläche der inneren Wandung aufgeklebt sein.

Des Weiteren können die Ausstülpungen auch mit Pelotten gefüllt sein.

Um eine oder mehrere Ausstülpungen nach Fertigstellung des Schafts der Prothese oder Orthese noch umpositionieren zu können, können die Ausstülpungen auch mobil oder temporär adaptierbar an der inneren Wandung angebracht sein.

Eine weitere vorteilhafte Ausgestaltung der Erfindung sieht die Integration eines TENS-Geräts zur Schmerzbehandlung an den Extremitäten in die Prothese oder Orthese vor. Die TENS-Geräte geben über Elektroden einen Reizstrom an die Haut ab. Eine solche TENS-Elektrode kann beispielsweise in einer Kammer einer Ausstülpung angeordnet sein. Das Therapieziel ist die Schmerzreduktion durch Verringerung oder Verhinderung der Schmerzweiterleitung an das Gehirn. Ein derartiges TENS-Gerät wird beispielsweise bei der Behandlung des Phantomschmerzes angewandt.

Eine weitere vorteilhafte Erfindung sieht vor, dass mindestens eine Kammer mindestens einer Ausstülpung einen Sensor für eine funktionelle Elektrostimulation, z.B. zur Behandlung nach einem Schlaganfall, aufweist.

Eine besonders vorteilhafte Ausgestaltung der Erfindung kann auch darin bestehen, mindestens eine der Ausstülpungen oder mindestens eine Kammer mindestens einer der Ausstülpungen mit verschiedenen Funktionen auszustatten. So kann mindestens eine Ausstülpung oder mindestens eine Kammer mindestens einer der Ausstülpungen eine Kombination der folgenden Merkmale aufweisen: einen Mechanismus zur Regulierung der Tiefe und/oder Dicke der Ausstülpungen, einen Permanentmagneten oder Elektromagneten, ein Piezoelement, ein Medikamentendepot, eine Heizung und/oder Kühlung, ein Vibrationselement, eine Füllung mit Feuchtigkeit aufsaugendem Material, ein Entfeuchtungselement, ein Messgerät zur Messung von Körperzuständen, ein Messgerät zur Messung von bionischen Daten, eine Elektrode zur Abnahme von myoelektrischen Impulsen, einen Temperaturlogger, einen Bewegungslogger, eine TENS-Elektrode. Dies ermöglicht eine gleichzeitige Behandlung verschiedener Beeinträchtigungen.

Für den Fall, dass die Ausstülpungen der Prothese oder Orthese für eine funktionelle Elektrostimulation ausgebildet sind, kann das Verfahren weiter dahingehend ausgestaltet sein, dass die Prothese oder Orthese ein elektrisches, mechanisches und/oder akustisches Signal aussendet, wenn die Prothese oder Orthese eine Bewegung des Körperteils registriert.

Des Weiteren umfasst die Verwendung der erfindungsgemäßen Prothese oder Orthese ihr Gebrauch als Arm- oder Beinprothese, als Arm- oder Beinorthese oder als Rumpfkorsett.

Nachfolgend werden einige vorteilhafte Ausführungsbeispiele einer erfindungsgemäßen Prothese oder Orthese anhand von Figuren genauer beschrieben. Die beschriebenen Merkmale sind jedoch nicht nur in Kombination der offenbarten Ausführungsbeispiele möglich, sondern können auch unabhängig vom konkreten Ausführungsbeispiel in verschiedenen Kombinationen realisiert sein. Es zeigen
- Figur 1: eine Seitenansicht einer Form für die Ausformung eines Beinprothesenschafts mit vier Vertiefungen,
- Figur 2: eine Seitenansicht einer Form für die Ausformung eines Beinprothesenschafts mit vier Auskragungen,
- Figur 3: eine Seitenansicht einer Form für die Ausformung eines Beinprothesenschafts mit mehreren Auskragungen,
- Figur 4: eine Seitenansicht einer Form für die Ausformung eines Beinprothesenschafts mit mehreren Vertiefungen,
- Figur 5: einen einschaligen Prothesenschaft mit einer Einrichtung zur hydraulischen Regulierung der Tiefe der Auskragungen,
- Figur 6: einen mehrschaligen Prothesenschaft mit einer Einrichtung zur hydraulischen Regulierung der Tiefe der Auskragungen,
- Figur 7: einen Prothesenschaft mit einer Einrichtung zur mechanischen Regulierung der Tiefe der Auskragungen,
- Figur 8: einen Prothesenschaft mit einem TENS-Gerät und
- Figur 9: eine schematische transparente Darstellung eines Oberschenkelstumpfes, in der der Ansatz einer Wärme- bzw. Kühlkammer gekennzeichnet ist.

Im Folgenden verweisen gleiche oder ähnliche Bezugszeichen auf gleiche oder ähnliche Elemente in den Figuren.

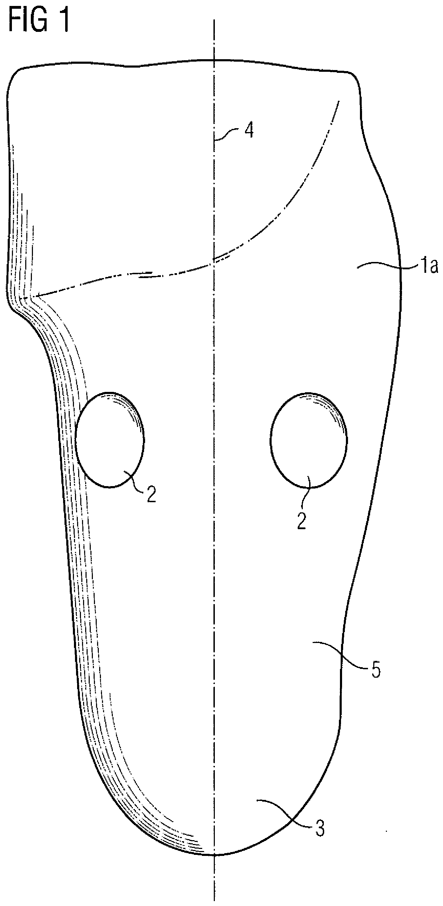

Figur 1 zeigt eine Seitenansicht einer Form 1a für die Ausformung einer inneren Wandung eines Schafts für eine Beinprothese mit vier Vertiefungen. Die Form 1a ist konisch ausgebildet und weist einen sich verjüngenden Teil 3 im unteren Bereich bezüglich einer Längsachse 4 der Form 1a auf. Im mittleren Bereich bezüglich der Längsachse 4 der Form 1a sind entlang eines Umfangs der Form 1a jeweils zueinander maximal beabstandet vier kuppelförmige Auskragungen 2 angeordnet. Als Umfang wird hier eine geschlossene Kurve bezeichnet, die eine Schnittfläche der Form 1a senkrecht zur Längsachse 4 einschließt.

Die Gestalt und Größe der Form 1a entspricht der Gestalt und Größe eines Oberschenkelstumpfes, der von dem Beinprothesenschaft (hier nicht gezeigt) aufgenommen werden kann. Zur Ausformung der inneren Wandung wird ein Schaftmaterial außen auf die Form 1a aufgebracht, sodass sich nach Entfernen der Form 1a ein zylindrischer Schaft (hier nicht gezeigt) mit einer konischen Ausnehmung in Gestalt der Form 1a ergibt. Diese konische Ausnehmung bildet einen Stumpfaufnahmebereich, dessen sich verjüngender Teil 3 der Prothese zugewandt ist. Der Stumpfaufnahmebereich wird von einer inneren Wandung des Schafts begrenzt. Die dem Stumpfaufnahmebereich zugewandte Oberfläche der inneren Wandung weist eine zur Reliefstruktur der Form 1a komplementäre Reliefstruktur auf. So entsprechen die kuppelförmigen Auskragungen 2 der Mantelfläche 5 der Form 1a komplementären kuppelförmigen Vertiefungen in der dem Stumpfaufnahmebereich zugewandten Oberfläche der inneren Wandung.

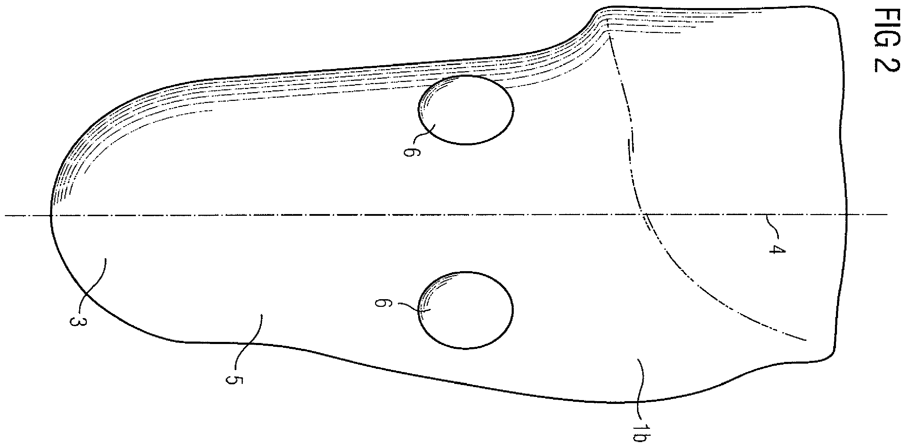

Figur 2 zeigt eine Seitenansicht einer weiteren Form 1b für die Ausformung einer inneren Wandung eines Schafts für eine Beinprothese mit vier Auskragungen. Die Form 1b ist ähnlich wie die Form 1a in Figur 1 ausgebildet. Die Form 1b unterscheidet sich lediglich dadurch von der Form 1a, dass die Mantelfläche 5 der Form 1b in ihrem mittleren Bereich bezüglich der Längsachse 4 der Form 1b anstatt kuppelförmiger Auskragungen 2 vier maximal zueinander beabstandet entlang eines Umfangs der Form 1b angeordnete kuppelförmige Vertiefungen 6 aufweist. Die Reliefstruktur der Mantelfläche 5 der Form 1b ist komplementär zur Reliefstruktur einer einem Stumpfaufnahmebereich zugewandten Oberfläche einer mittels der Form 1b ausgeformten inneren Wandung eines Beinprothesenschafts (hier nicht gezeigt). So entsprechen die kuppelförmigen Vertiefungen 6 in der Mantelfläche 5 der Form 1b komplementären kuppelförmigen Auskragungen in einer einem Stumpfaufnahmebereich zugewandten Oberfläche einer mittels der Form 1b ausgeformten inneren Wandung eines Beinprothesenschafts.

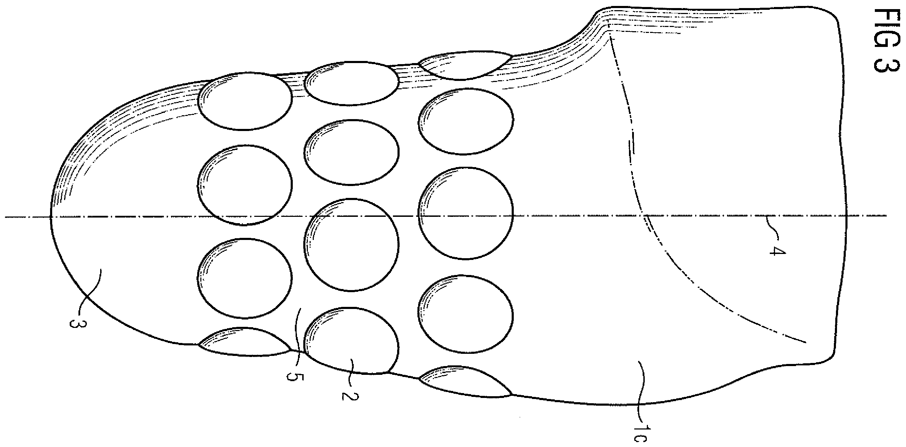

Figur 3 zeigt eine Seitenansicht einer weiteren Form 1c für die Ausformung einer inneren Wandung eines Schafts für eine Beinprothese mit einer Vielzahl von Vertiefungen. Die Form 1c ist ähnlich wie die Formen 1a und 1b in den Figuren 1 und 2 ausgebildet. Die Form 1c unterscheidet sich lediglich dadurch von den Formen 1a und 1b, dass die Mantelfläche 5 der Form 1c in ihrem mittleren Bereich bezüglich der Längsachse 4 der Form 1c in drei Reihen entlang eines Umfangs der Form 1c angeordnete kuppelförmige Auskragungen 2 aufweist. Dabei sind die Auskragungen 2 bezüglich einer Richtung parallel zur Längsachse 4 versetzt zueinander angeordnet. Die Reliefstruktur der Mantelfläche 5 der Form 1c ist komplementär zur Reliefstruktur einer einem Stumpfaufnahmebereich zugewandten Oberfläche einer mittels der Form 1c ausgeformten inneren Wandung eines Beinprothesenschafts (hier nicht gezeigt). So entsprechen die kuppelförmigen Auskragungen 2 in der Mantelfläche 5 der Form 1c komplementären kuppelförmigen Vertiefungen in einer einem Stumpfaufnahmebereich zugewandten Oberfläche einer mittels der Form 1c ausgeformten inneren Wandung eines Beinprothesenschafts.

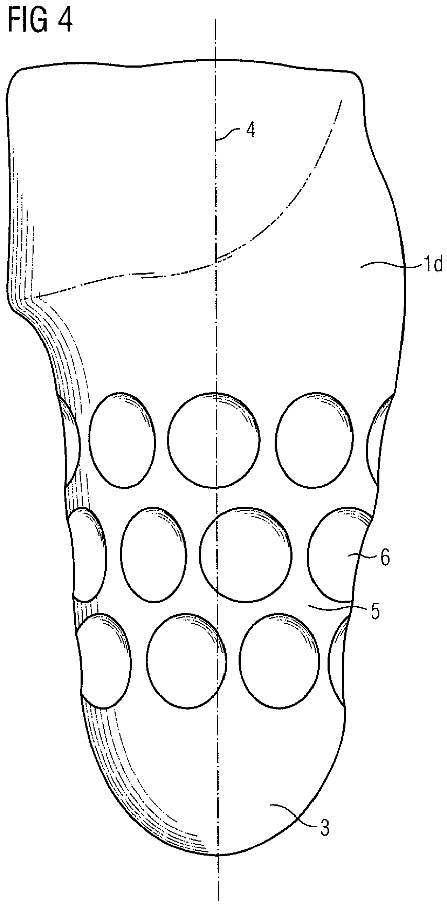

Figur 4 zeigt eine Seitenansicht einer weiteren Form 1d für die Ausformung einer inneren Wandung eines Schafts für eine Beinprothese mit einer Vielzahl von Auskragungen. Die Form 1d ist ähnlich wie die Formen 1a, 1b und 1c in den Figuren 1, 2 und 3 ausgebildet. Die Form 1d unterscheidet sich lediglich dadurch von den Formen 1a, 1b und 1c, dass die Mantelfläche 5 der Form 1d in ihrem mittleren Bereich bezüglich der Längsachse 4 der Form 1d in drei Reihen entlang eines Umfangs der Form 1d angeordnete kuppelförmige Vertiefungen 6 aufweist. Dabei sind die Vertiefungen 6 bezüglich einer Richtung parallel zur Längsachse 4 versetzt zueinander angeordnet. Die Reliefstruktur der Mantelfläche 5 der Form 1d ist komplementär zur Reliefstruktur einer einem Stumpfaufnahmebereich zugewandten Oberfläche einer mittels der Form 1d ausgeformten inneren Wandung eines Beinprothesenschafts (hier nicht gezeigt). So entsprechen die kuppelförmigen Vertiefungen 6 in der Mantelfläche 5 der Form 1d komplementären kuppelförmigen Auskragungen in einer einem Stumpfaufnahmebereich zugewandten Oberfläche einer mittels der Form 1d ausgeformten inneren Wandung eines Beinprothesenschafts.

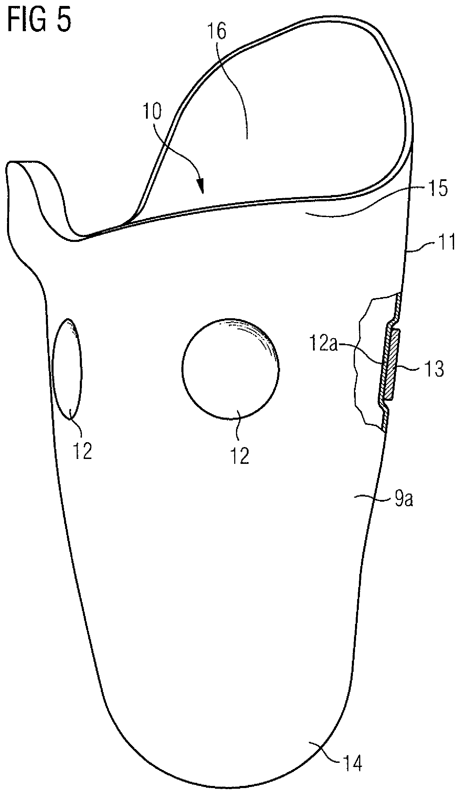

Figur 5 zeigt einen einschaligen Prothesenschaft 9a mit vier Auskragungen in einer Perspektivansicht. Der Prothesenschaft 9a weist eine hohle, konische Form auf und ist zur Aufnahme eines Oberschenkelstumpfes geeignet. Der Prothesenschaft 9a weist weiter eine Prothesenschaftwand 11 auf. Auf einer einer sich verjüngend zulaufenden Seite 14 abgewandten Seite 15 des Prothesenschafts 9a weist der Prothesenschaft 9a eine Öffnung 10 auf, von der an sich der Stumpfaufnahmebereich 16 bis zur sich verjüngend zulaufenden Seite 14 des Prothesenschafts 9a und innerhalb der Prothesenschaftwand 11 erstreckt. Somit weist der Prothesenschaft 9a eine ebenfalls konisch geformte Ausnehmung als Stumpfaufnahmebereich 16 auf. Im oberen Bereich des Prothesenschafts 9a, das heißt, in dem Bereich, der der Öffnung 10 zugewandt ist, weist die Prothesenschaftwand 11 vier entlang des Umfangs des Prothesenschafts 9a und zum Stumpfaufnahmebereich 16 hin gerichtete Auskragungen 12 auf. Eine der Auskragungen 12, Auskragung 12a, weist eine Einrichtung 13 zur hydraulischen Regulierung der Tiefe der Auskragung 12a auf. Mit Tiefe ist hier das Maß gemeint, in welchem die Auskragung 12a in den Stumpfaufnahmebereich 16 hineinragt. Die Einrichtung 13 ist nicht von der Prothesenschaftwand 11 überdeckt sondern von außen sichtbar und zugänglich. Bei Betätigen der Einrichtung 13 wölbt sich die Einrichtung 13 mehr oder weniger in Richtung des Stumpfaufnahmebereichs 16 und vergrößert oder verringert dadurch die Tiefe der Auskragung 12a.

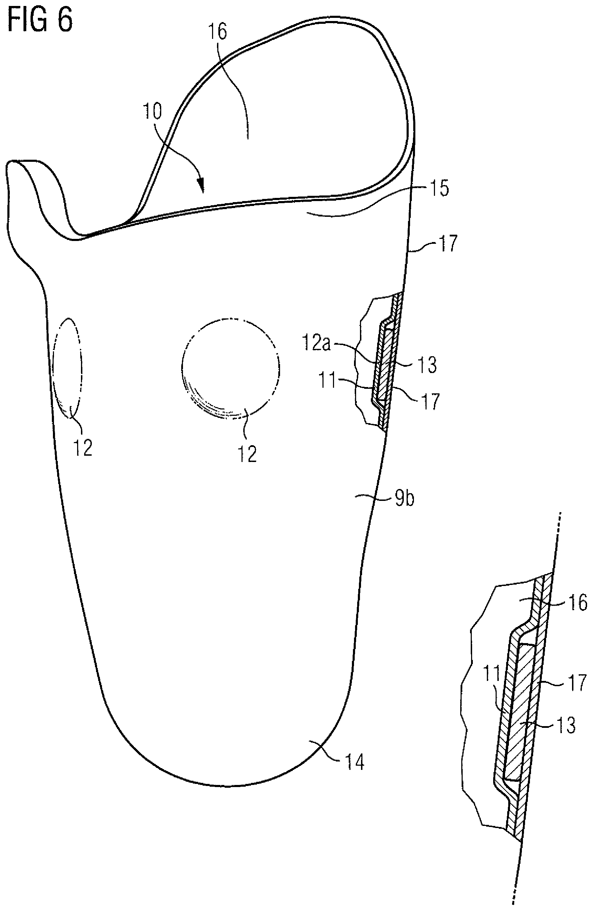

Figur 6 zeigt einen zweischaligen Prothesenschaft 9b, der ähnlich zu dem Prothesenschaft 9b aus Figur 5 ausgebildet ist und ebenfalls vier Auskragungen 12 aufweist, wobei die Auskragung 12a eine Einrichtung 13 zur hydraulischen Regulierung der Tiefe der Auskragung 12a beinhaltet. Im weiteren Unterschied zum Prothesenschaft 9a der Figur 5 weist der Prothesenschaft 9b eine zweischalige Prothesenschaftwand auf, wobei diese zum Stumpfaufnahmebereich 16 hin eine innere Wandung 11 und nach außen hin bzw. vom Stumpfaufnahmebereich 16 weg gerichtet eine äußere Wand 17 aufweist. Dementsprechend ist die Einrichtung 13 zur hydraulischen Regulierung der Tiefe der Auskragung 12a zwischen der inneren Wandung 11 und der äußeren Wand 17 angeordnet, sodass die Einrichtung 13 von außen weder sichtbar noch zugänglich ist.

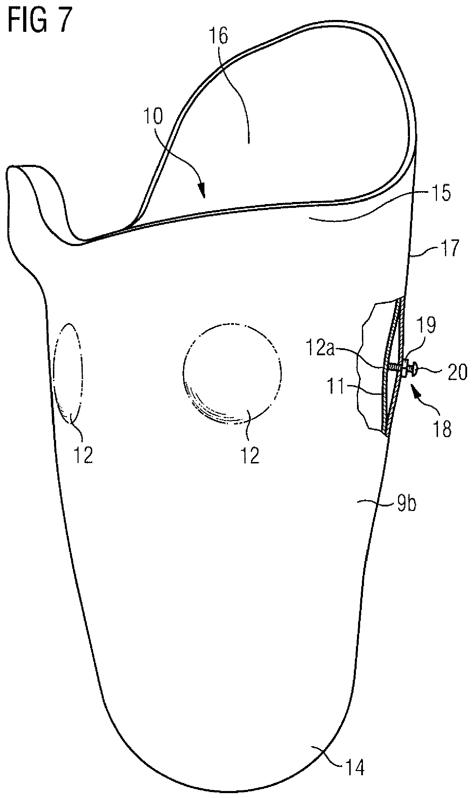

Figur 7 zeigt den Prothesenschaft 9b aus Figur 4, wobei dieser anstatt einer Einrichtung 13 zur hydraulischen Regulierung nun eine Einrichtung 18 zur mechanischen Regulierung der Tiefe der Auskragung 12a aufweist. Die Einrichtung 18 weist eine in etwa senkrecht zur inneren Wandung 11 und zur äußeren Wand 17 in ein Gewinde 19 eingeschraubte Schraube 20 auf. Das Gewinde 19 ist in der äußeren Wand 17 enthalten, sodass der Kopf der Schraube 20 nach außen gerichtet ist und von außen zur Betätigung der 20 zugänglich ist. Das dem Kopf der Schraube 20 abgewandte Ende der Schraube 20 berührt die innere Wandung 11 auf der dem Stumpfaufnahmebereich 16 abgewandten Seiten der inneren Wandung 11. Durch Ein- und Ausdrehen der Schraube 20 wird die innere Wandung 11 tiefer oder weniger tief zum Stumpfaufnahmebereich 16 hin gedrückt und somit die Tiefe der Auskragung vergrößert bzw. verkleinert.

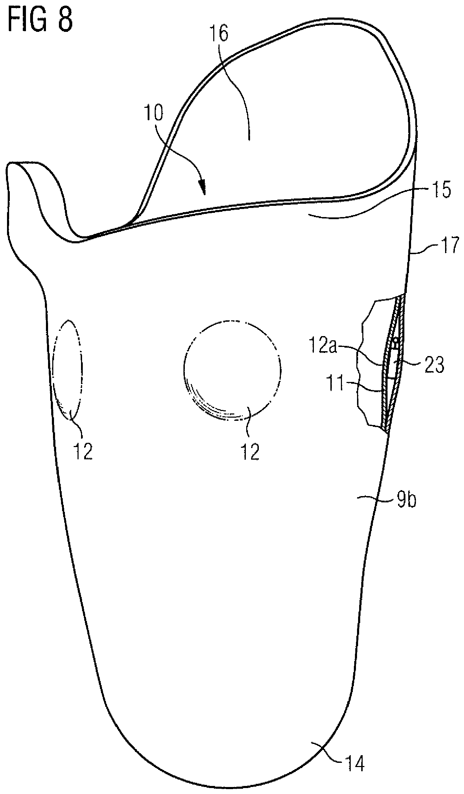

Figur 8 zeigt den Prothesenschaft 9b, wobei die Auskragung 12a ein TENS-Gerät 23 aufweist. Auch hier ist das TENS-Gerät 23 zwischen der inneren Wandung 11 und der äußeren Wand 17 angeordnet.

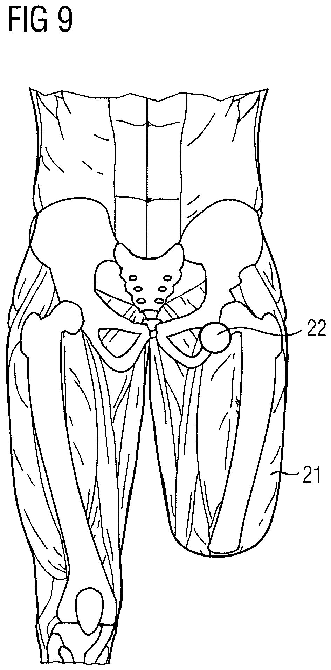

Figur 9 zeigt eine transparente schematische Darstellung eines menschlichen Oberschenkelstumpfes 21. Der Kreis 22 im oberen Bereich des dargestellten Oberschenkelstumpfes 21 kennzeichnet einen Ansatzpunkt für eine Wärme- und/oder Kühlkammer, die in einer Ausstülpung enthalten ist. Bei dem dargestellten Ansatzpunkt handelt es sich um eine zentrale Einströmstelle des arteriellen Blutes. Wird das Blut an dieser Stelle erwärmt bzw. abgekühlt, kann sich die Wärme bzw. Kühle aufgrund des Blutflusses schnell im gesamten Oberschenkelstumpf verteilen.

In einem weiteren Aspekt der vorliegenden Erfindung kann mindestens eine der Ausstülpungen der Prothese oder Orthese mindestens eine Kammer aufweisen.

In einem weiteren Aspekt der vorliegenden Erfindung kann mindestens eine Kammer mindestens einer der Ausstülpungen einen Permanentmagneten oder einen Elektromagneten zur Erzeugung eines Magnetfeldes beinhalten.

In einem weiteren Aspekt der vorliegenden Erfindung kann mindestens eine Kammer mindestens einer der Ausstülpungen ein Piezoelement zur elektrischen und/oder mechanischen Stimulation beinhalten.

In einem weiteren Aspekt der vorliegenden Erfindung können in mindestens einer Kammer mindestens einer der Ausstülpungen Medikamente deponierbar sein, wobei die Medikamente über eine oder mehrere Öffnungen und/oder Poren in der mindestens einen Kammer an eine Hautoberfläche des Körperteils abgebbar sind.

In einem weiteren Aspekt der vorliegenden Erfindung können mindestens eine der Ausstülpungen zur Beheizung und/oder Kühlung des Schaftes eingerichtet sein.

In einem weiteren Aspekt der vorliegenden Erfindung kann der Schaft von einem Schlauchsystem zur Heizung und/oder Kühlung durchzogen sein, das mit mindestens einer der Ausstülpungen wärmeleitend verbunden ist.

In einem weiteren Aspekt der vorliegenden Erfindung kann mindestens eine Kammer mindestens einer der Ausstülpungen als Wärmekammer zur Erwärmung von arteriellem Blut und /oder einer Kühlkammer zur Abkühlung arteriellen Blutes an einer zentralen Einströmstelle des arteriellen Blutes eingerichtet sein.

In einem weiteren Aspekt der vorliegenden Erfindung kann mindestens eine Kammer mindestens einer der Ausstülpungen ein Vibrationselement beinhalten.

In einem weiteren Aspekt der vorliegenden Erfindung kann mindestens eine Kammer mindestens einer der Ausstülpungen eine Füllung mit Feuchtigkeit aufsaugendem Material aufweisen.

In einem weiteren Aspekt der vorliegenden Erfindung kann in mindestens einer Kammer mindestens einer der Ausstülpungen ein elektronisch und/oder mechanisch wirkendes Entfeuchtungselement angeordnet sein.

In einem weiteren Aspekt der vorliegenden Erfindung kann in mindestens einer Kammer mindestens einer der Ausstülpungen mindestens ein Messgerät zur Messung von Körperzuständen, eines Blutdrucks, einer Strömungsgeschwindigkeit von Blut und/oder einer Körpertemperatur angeordnet sein.

In einem weiteren Aspekt der vorliegenden Erfindung kann in mindestens einer Kammer mindestens einer der Ausstülpungen mindestens ein Messgerät zur Erfassung von bionischen Daten, eines Oberflächendruckes an definierten Messpunkten, einer Schaftinnentemperatur und/oder eines Feuchtigkeitsgrades angeordnet sein.

In einem weiteren Aspekt der vorliegenden Erfindung kann mindestens eine der Ausstülpungen mindestens eine Elektrode zur Abnahme von myoelektrischen Impulsen aufweisen.

In einem weiteren Aspekt der vorliegenden Erfindung kann mindestens eine der Ausstülpungen mindestens einen Temperatur- und/oder Bewegungslogger aufweisen.

In einem weiteren Aspekt der vorliegenden Erfindung kann mindestens eine der Ausstülpungen und die innere Wandung unterschiedliche Materialien aufweisen.

In einem weiteren Aspekt der vorliegenden Erfindung kann die innere Wandung ein elastisches Material, ein Material mit einem weichen Shore-Grad oder ein rigides Material aufweisen, in das die Ausstülpungen integriert sind. In einem weiteren Aspekt der vorliegenden Erfindung kann eine Oberfläche mindestens einer der Ausstülpungen glatt, rau, adhäsiv, mit einem Relief und/oder mit Noppen versehen sein.

In einem weiteren Aspekt der vorliegenden Erfindung können die Noppen vollständig mit Material gefüllt oder hohl gestaltet sein.

In einem weiteren Aspekt der vorliegenden Erfindung kann mindestens eine der Ausstülpungen auf eine Oberfläche der inneren Wandung aufgeklebt sein. In einem weiteren Aspekt der vorliegenden Erfindung kann mindestens eine der Ausstülpungen mit einer Pelotte gefüllt sein.

In einem weiteren Aspekt der vorliegenden Erfindung kann mindestens eine Kammer mindestens einer der Ausstülpungen mindestens eine TENS-Elektrode aufweisen.

In einem weiteren Aspekt der vorliegenden Erfindung kann mindestens eine Kammer mindestens einer der Ausstülpungen einen Sensor für eine funktionelle Elektrostimulation aufweisen.

## Patentansprüche

1. Prothese oder Orthese mit einem mehrschaligen oder einschaligen Schaft (9a, 9b) zur Aufnahme eines Körperteils, wobei der Schaft (9a, 9b) eine einen Körperteilaufnahmebereich (16) begrenzende innere Wandung (11) aufweist, wobei die innere Wandung (11) den Körperteilaufnahmebereich (16) in einer Ebene vollständig umschließt und wobei die innere Wandung (11) mindestens zwei Ausstülpungen (12) aufweist,
**dadurch gekennzeichnet,**
**dass** mindestens eine der Ausstülpungen (12) temporär adaptierbar an der inneren Wandung (11) angebracht ist, sodass die mindestens eine der Ausstülpungen (12) umpositionierbar ist.

2. Prothese oder Orthese nach dem vorhergehenden Anspruch, **dadurch gekennzeichnet, dass** die innere Wandung (11) mindestens vier Ausstülpungen (12) aufweist, wobei die Ausstülpungen (12) derart angeordnet sind, dass in einer Projektion der inneren Wandung (11) auf eine Ebene senkrecht zu einer Längsachse des Schafts (9a, 9b) die Ausstülpungen (12) lediglich bereichsweise überlappen.

3. Prothese oder Orthese nach einem der vorhergehenden Ansprüche, **dadurch gekennzeichnet, dass** mindestens eine der Ausstülpungen (12) als Auskragung (12) oder als Vertiefung ausgebildet ist.

4. Prothese oder Orthese nach einem der vorhergehenden Ansprüche, **dadurch gekennzeichnet, dass** mindestens eine der Ausstülpungen (12) kuppelförmig, kantig, konvex, konkav, sternförmig, oval oder eckig ausgebildet sind.

5. Prothese oder Orthese nach einem der vorhergehenden Ansprüche, **dadurch gekennzeichnet, dass** der Schaft (9a, 9b) zylinderförmig oder konisch gestaltet ist.

6. Prothese oder Orthese nach einem der vorhergehenden Ansprüche, **dadurch gekennzeichnet, dass** eine Tiefe und/oder eine Dicke mindestens einer der Ausstülpungen (12) pneumatisch oder hydraulisch oder mechanisch veränderbar ist.

7. Prothese oder Orthese nach dem vorhergehenden Anspruch, **dadurch gekennzeichnet, dass** im Schaft (9a, 9b) ein System miteinander kommunizierender Röhren zur hydraulischen oder pneumatischen oder mechanischen Regulierung der Tiefe und/oder Dicke der Ausstülpungen (12) angeordnet ist.

8. Prothese oder Orthese nach einem der beiden vorhergehenden Ansprüche, **dadurch gekennzeichnet, dass** zur Regulierung der Tiefe und/oder der Dicke der Ausstülpungen (12) ein Wechseldrucksystem vorgesehen ist, womit die Tiefe und/oder Dicke von einzelnen oder mehreren Ausstülpungen (12) abwechselnd erhöht oder verringert werden kann.

9. Prothese oder Orthese nach einem der vorhergehenden Ansprüche, **dadurch gekennzeichnet, dass** mindestens eine der Ausstülpungen (12) mindestens eine Kammer aufweist.

10. Prothese oder Orthese nach dem vorhergehenden Anspruch, **dadurch gekennzeichnet, dass** mindestens eine Kammer mindestens einer der Ausstülpungen (12) einen Permanentmagneten oder einen Elektromagneten zur Erzeugung eines Magnetfeldes beinhaltet.

11. Prothese oder Orthese nach einem der Ansprüche 9 oder 10, **dadurch gekennzeichnet, dass** mindestens eine Kammer mindestens einer der Ausstülpungen (12) ein Piezoelement zur elektrischen und/oder mechanischen Stimulation beinhaltet.

## Claims

1. A prosthesis or orthosis with a multi-shell or single-shell shaft (9a, 9b) for receiving a body part, wherein the shaft (9a, 9b) has an inner wall (11) delimiting a body-part receiving region (16), wherein the inner wall (11) completely encloses the body-part receiving region (16) in one plane and wherein the inner wall (11) has at least two protuberances (12),
**characterised in that** at least one of the protuberances (12) is attached temporarily in adaptable manner to the inner wall (11), so that the at least one of the protuberances (12) is repositionable.

2. A prosthesis or orthosis according to the preceding claim, **characterised in that** the inner wall (11) has at least four protuberances (12), the protuberances (12) being arranged such that in a projection of the inner wall (11) onto a plane perpendicular to a longitudinal axis of the shaft (9a, 9b) the protuberances (12) overlap merely in regions.

3. A prosthesis or orthosis according to one of the preceding claims, **characterised in that** at least one of the protuberances (12) is formed as a projection (12) or as an indentation.

4. A prosthesis or orthosis according to one of the preceding claims, **characterised in that** at least one of the protuberances (12) is/are designed in a dome-shape, in edged, convex, concave, star-shaped or oval manner, or with corners.

5. A prosthesis or orthosis according to one of the preceding claims, **characterised in that** the shaft (9a, 9b) is configured to be cylindrical or conical.

6. A prosthesis or orthosis according to one of the preceding claims, **characterised in that** a depth and/or a thickness of at least one of the protuberances (12) is changeable pneumatically or hydraulically or mechanically.

7. A prosthesis or orthosis according to the preceding claim, **characterised in that** a system of communicating tubes for hydraulic or pneumatic or mechanical regulation of the depth and/or thickness of the protuberances (12) is arranged in the shaft (9a, 9b).

8. A prosthesis or orthosis according to one of the preceding two claims, **characterised in that** an alternating-pressure system is provided for regulating the depth and/or the thickness of the protuberances (12), with which system the depth and/or thickness of individual or a plurality of protuberances (12) can be increased or reduced in alternation.

9. A prosthesis or orthosis according to one of the preceding claims, **characterised in that** at least one of the protuberances (12) has at least one chamber.

10. A prosthesis or orthosis according to the preceding claim, **characterised in that** at least one chamber of at least one of the protuberances (12) contains a permanent magnet or an electromagnet for generating a magnetic field.

11. A prosthesis or orthosis according to one of Claims 9 or 10, **characterised in that** at least one chamber of at least one of the protuberances (12) contains a piezo element for electrical and/or mechanical stimulation.

## Revendications

1. Prothèse ou orthèse avec une tige multicoque ou monocoque (9a, 9b) pour le logement d'un membre, la tige (9a, 9b) comprenant une paroi interne (11) délimitant une partie de logement de membre (16), la paroi interne (11) entourant complètement la partie de logement de membre (16) dans un plan et la paroi interne (11) comprenant au moins deux protubérances (12),
**caractérisée en ce que**
au moins une des protubérances (12) peut être montée de manière temporairement adaptable sur la paroi interne (11), de façon à ce que l'au moins une des protubérances (12) puisse être déplacée.

2. Prothèse ou orthèse selon la revendication précédente, **caractérisée en ce que** la paroi interne (11) comprend au moins quatre protubérances (12), les protubérances (12) étant disposées de façon à ce que, dans une projection de la paroi interne (11) sur un plan perpendiculaire à un axe longitudinal de la tige (9a, 9b), les protubérances (12) se superposent seulement partiellement.

3. Prothèse ou orthèse selon l'une des revendications précédentes, **caractérisée en ce qu'**au moins une des protubérances (12) est conçue comme une saillie (12) ou comme un creux.

4. Prothèse ou orthèse selon l'une des revendications précédentes, **caractérisée en ce qu'**au moins une des protubérances (12) présente une forme de coupole, une forme d'étoile, une forme anguleuse, convexe, concave, ovale ou angulaire.

5. Prothèse ou orthèse selon l'une des revendications précédentes, **caractérisée en ce que** la tige (9a, 9b) présente une forme cylindrique ou conique.

6. Prothèse ou orthèse selon l'une des revendications précédentes, **caractérisée en ce que** la profondeur et/ou l'épaisseur d'au moins une des protubérances (12) peut être modifiée de manière pneumatique, hydraulique ou mécanique.

7. Prothèse ou orthèse selon la revendication précédente, **caractérisée en ce que**, dans la tige (9a, 9b), est disposé un système de tubes communiquant entre eux pour la régulation hydraulique, pneumatique ou mécanique de la profondeur et/ou de l'épaisseur des protubérances (12).

8. Prothèse ou orthèse selon l'une des deux revendications précédentes, **caractérisée en ce que**, pour la régulation de la profondeur et/ou de l'épaisseur des protubérances (12), un système de pression variable est prévu, qui permet tout à tour d'augmenter ou de réduire la profondeur et/ou l'épaisseur de certaines ou de plusieurs protubérances (12).

9. Prothèse ou orthèse selon l'une des revendications précédentes, **caractérisée en ce qu'**au moins une des protubérances (12) comprend au moins une chambre.

10. Prothèse ou orthèse selon la revendication précédente, **caractérisée en ce qu'**au moins une chambre d'au moins une des protubérances (12) contient un aimant permanent ou un électro-aimant pour la production d'un champ magnétique.

11. Prothèse ou orthèse selon l'une des revendications 9 ou 10, **caractérisée en ce que** l'au moins une chambre d'au moins une des protubérances (12) contient un élément piézo-électrique pour la stimulation électrique et/ou mécanique.
